(19) Europäisches Patentamt
European Patent Office
Office européen des brevets

(11) **EP 4 520 352 A1**

(12) **EUROPEAN PATENT APPLICATION**

(43) Date of publication:
**12.03.2025 Bulletin 2025/11**

(21) Application number: **24172723.9**

(22) Date of filing: **26.04.2024**

(51) International Patent Classification (IPC):
**A61L 2/10** (2006.01)    **A61L 2/26** (2006.01)

(52) Cooperative Patent Classification (CPC):
**A61L 2/10; A61L 2/26;** A61L 2202/11;
A61L 2202/16; A61L 2202/23

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.04.2023 US 202363498360 P
22.12.2023 US 202363614053 P**

(71) Applicant: **P Tech, LLC
Manalapan, FL 33462 (US)**

(72) Inventors:
• **Bonutti, Peter M.
Manalapan, 33462 (US)**
• **Beyers, Justin E.
Effingham, 62401 (US)**
• **Kurmann, Barrett
Effingham, 62401 (US)**

(74) Representative: **Cyrson, Matthew Dominic et al
Albright IP Limited
County House
Bayshill Road
Cheltenham, Glos. GL50 3BA (GB)**

(54) **ADAPTER FOR COUPLING UV DISINFECTION DEVICE TO CONTAINER**

(57)     An adapter couples an ultraviolet (UV) disinfecting device to a container (208) suitable for holding liquid. The adapter may be a lid adapter (200) that also functions as a lid for the container (208). The adapter includes a body (202) and a coupler (204). The body (202) couples to the UV disinfecting device, and the coupler (204) couples to the container (208). In this way, the UV disinfecting device is in communication with the interior of the container (208).

FIG. 1

EP 4 520 352 A1

**Description**

FIELD OF THE DISCLOSURE

The present disclosure generally relates to an adapter for coupling a UV disinfection device to a container.

BACKGROUND OF THE DISCLOSURE

It may be necessary to detect pathogens or illness. It may be necessary to prevent illnesses, such as illnesses caused by pathogens or other abnormal cells/tissues. It may be necessary to treat objects, surfaces, an organism (e.g., human or animal), a plant, or other things to kill pathogens, kill cells, or otherwise treat. It may also be necessary to protect against pathogens which may infect an individual, especially in a time of widespread interaction between people.
A common problem with reusable water bottles, faced by consumers, is the rapid growth of bacteria colonies. A study done at Henan University shows that the average adult's water bottle contains 75,000 bacteria counts/mL.

SUMMARY OF DISCLOSURE

In one aspect, an adapter for coupling an ultraviolet (UV) disinfecting device to a container suitable for holding liquid generally comprises an adapter body configured to enable the UV disinfecting device to be removably coupled thereto, the adapter body defining a window; and an adapter coupler connected to the adaptor body, the coupler configured to removably couple with the container to thereby removably couple the adapter to the container. When the UV disinfecting device is coupled to the adapter body and the adapter coupler is coupled to the container, the window of the adaptor body is alignable with a UV light source of the disinfecting device and configured to enable the UV light source to be in communication with the interior of the container.
In another aspect, a lid adapter for coupling an ultraviolet (UV) disinfecting device to a container suitable for holding liquid generally comprises an adapter body configured to enable the UV disinfecting device to be removably coupled thereto, the adapter body defining a window; and an adapter coupler connected to the adaptor body, the coupler configured to removably couple to an open top portion of the container to thereby removably couple the adapter to the container. When the UV disinfecting device is coupled to the adapter body and the adapter coupler is coupled to the container, the window of the adaptor body is alignable with a UV light source of the disinfecting device and configured to enable the UV light source to be in communication with the interior of the container.
Other aspects of the disclosure are described below.

BRIEF DESCRIPTION OF THE DRAWINGS

FIG. 1 is a schematic representation of a UVC device in communication with an external processor.

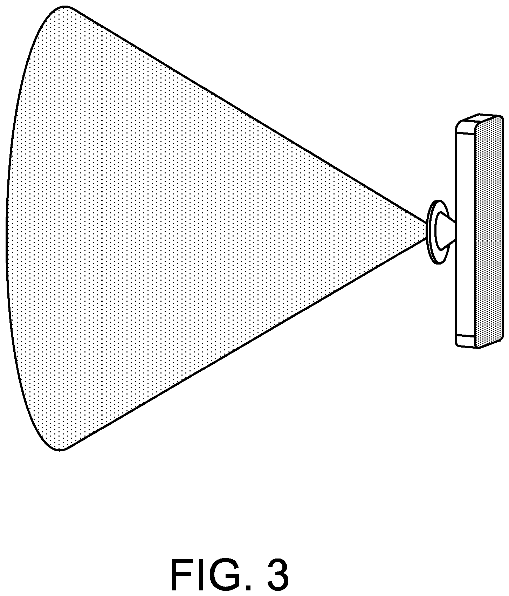

FIGS. 2-9 are schematic representations of the UVC device in combination with a hand-held device.
FIG. 10 is a front elevation of a personal protection face mask.
FIG. 11 is a cross section of the face mask.
FIG. 12 is a front elevation of another embodiment of a face mask.
FIG. 13 is a cross section of the face mask.

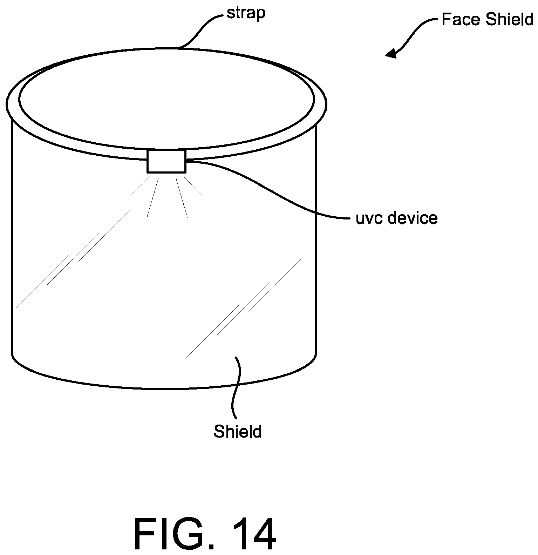

FIG. 14 is a front view of a face shield.

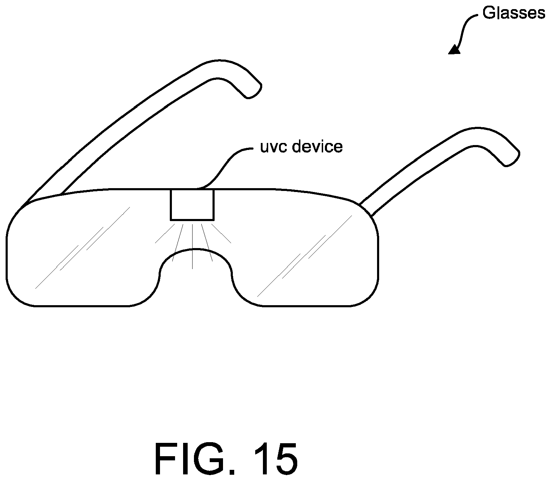

FIG. 15 is a front view of a pair of glasses.
FIG. 16 is a schematic representation of an endotracheal tube system;
FIG. 17 is a cross section of the endotracheal tube.
FIG. 18 is a perspective of a lid adapter for enabling a UV disinfectant device to be used on a hand-held, liquid container, such as a water bottle.
FIG. 19 is a side elevation of the lid adapter including UV disinfectant device docked and coupled to the lid adapter.
FIG. 20 is a front elevation of a suitable bottle for use with the lid adapter.
FIG. 21 is a suitable UV disinfection device for use with the lid adapter.
FIG. 22 is a top plan view of another embodiment of a lid adapter.
FIG. 23 is a perspective of another embodiment of a lid adapter.
FIG. 24 is a perspective of FIG. 19 showing the lid adapter and UV disinfection device coupled thereto.
FIG. 25 is a rear plan view of a strip of magnetic material.

DETAILED DESCRIPTION OF THE DISCLOSURE

### A. Systems and Methods for Personal Protection Against Pathogens

Systems and methods for personal protection against pathogens are shown and described throughout the drawings and below. These systems and methods are constructed according to the teachings of the present disclosure and are not limiting. Variations may be constructed based on the below teachings. Moreover, the teachings of one embodiment may be readily used in another embodiment and combined in any suitable manner to further provide person protection against pathogens.

In one such disclosure, described herein broadly as the "UVC Disinfection/Sterilization" disclosure, a UVC device is used to disinfect or substantially sterilize a person's environment or surroundings, such as but not limited to surrounding surfaces, fluids, and/or air. The source of UVC may include a light emitting diode (LED) or other UVC emitting source. The source of UVC may be controllable by a processor or control unit (broadly, a controller). For example, control of the UVC may be performed by a software application run by a processor (e.g., CPU) in communication (wireless or wired) with the UVC source (e.g., LED). The processor may be part of a mobile device (e.g., a hand-held mobile phone or tablet) or other device, such as a robot. Other functions for use in disinfecting/sterilization, as described below, may also be controlled by the processor. The UVC device may be combined with other disinfection/sterilization devices and methods or be the sole source of disinfection/sterilization. For example, the UVC made be combined with one or more of: filter media for filtering pathogens (e.g., a respirator or face mask), chemical disinfectants (e.g., alcohols, hydrogen peroxide, hydrogen peroxide vapor (HPV), among others).

In another disclosure, described herein broadly as the "Infection Risk Detection" disclosure-which may be combined with or separate from the UVC disclosure-the state of sterilization or cleanliness of a person's surroundings, including air, liquid, surfaces, and other persons, may be detected and analyzed using a sensor capable of detecting a parameter of the person's surroundings. The sensor may be in communication with a processor or control unit (broadly, a controller). For example, the sensor may generate a signal (digital or analog) indicative of the parameter of the person's surroundings. This signal is transmitted to and analyzed by the processor, such as by using software application. The processor may also in communication with the sensor for controlling the sensor. The processor may be part of a mobile device (e.g., a hand-held mobile phone or tablet) or other device, such as a robot. Other functions for use in detecting sterilization or cleanliness of a person's surroundings, as described below, may also be controlled by the processor. This Detection disclosure may be combined with other types of disinfection/sterilization systems other than UVC.

In yet another disclosure, described herein as the "Medical Device Disinfection/Sterilization" disclosure-which may be combined with or separate from one or both of the UVC and Detection disclosure-sterilization is performed inside a catheter, cannula, tubing, or other device defining an internal passage for the delivery of fluid, gas, a medical device, or other deliverables (broadly, a medical delivery device) which is insertable into a patient's body. The medical delivery device includes a sterilizer which is capable of sterilizing the delivered fluid, gas, and/or medical device, etc., inside the patient's body and within the medical delivery device. The type of sterilization is not necessarily limited and may include a combination of sterilization techniques.

In yet another disclosure, various other treatments and uses of UVC may be employed, as described below.

The following applications are incorporated by reference herein: US Serial No. 16/025,152, filed July 2, 2018; US Serial No. 16/113,666, filed August 18, 2018; US Serial No. 16/118,025, filed August 30, 2018; and US Serial No. 09/941,185, filed August 28, 2001.

### 1. UVC Disinfection/Sterilization

The following UVC disinfection/sterilization disclosure is divided into exemplary embodiments for ease of disclosure. Teachings in each embodiment apply equally to the other embodiments and may be combined therewith. The UVC may be wearable or donnable. The UVC device may be hand-held. The UVC device may be attachable to a hand-held device, such as a mobile electronic device (e.g., a mobile smartphone or tablet). The UVC device may be combined with other disinfection/sterilization devices and methods or be the sole source of disinfection/sterilization. For example, the UVC made be combined with one or more of: filter media for filtering pathogens (e.g., a respirator or face mask), chemical disinfectants (e.g., alcohols, hydrogen peroxide, hydrogen peroxide vapor (HPV), among others). The teachings set forth in US Serial No. 16/025,152 are also incorporated herein.

UVC in Communication with a Processor

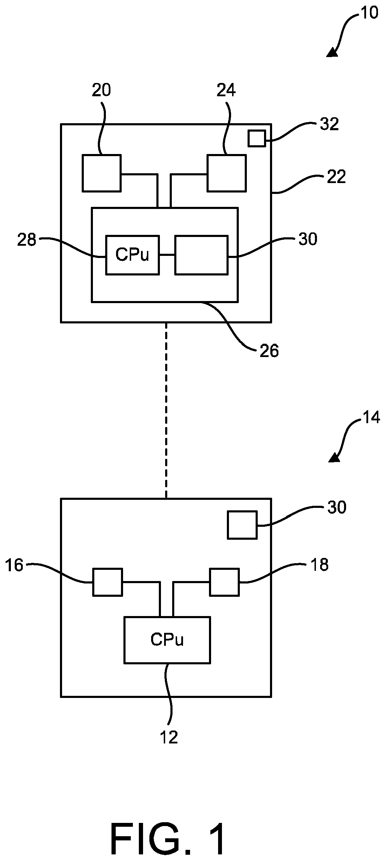

Referring to FIG. 1, in one exemplary embodiment, UVC device, generally indicated at 10, is in communication (wired or wireless) with an external processor 12. In this embodiment, the processor is part of another device 14 (e.g., a hand-held device), which also includes memory 16, a battery 18 or other power source, and other circuitry and components such as a communication device(s) (e.g., wireless transceivers) for communicating with the UVC device, as is generally known in the art. The UVC device includes a UVC source 20, such as an LED or other source capable of emitting UVC radiation. The UVC source may be coupled to or mounted on a housing 22 or other support structure. The UVC device may include electronics (e.g., a driver circuit) suitable for operating the UVC source. A power source 24 (e.g., battery) may be included in the support structure for powering the UVC source. A controller 26 (e.g., a processor 28 and memory 30) may also be present in the UVC device (e.g., in the housing) for controlling the UVC source. The UVC device may also include optics, such as a lens (Fresnel lens), collimator, collector, etc., may also be in communication with the UVC source. It is also considered that the UVC output could be transmitted through fiber optic filaments or a prism. The support structure may further be configured to be wearable or donnable or couplable to a hand-held device, as described in an example below. The UVC device may be integrated into another device, such as an electronic device. Such an electronic device may include a robot. The robot may be configured to control operation of the UVC source.

The external processor is capable of accessing a software application (or other software program) stored in memory. The application may comprise instructions for controlling the UVC source and other functions of the UVC device, such as explained in more detail below in exemplary embodiments. In one example, the processor is part of a hand-held, electronic mobile device, such as a smart phone or tablet. Memory including the software application accessible by the processor may also be part of the hand-held device. A camera 30 of the hand-held electronic mobile device may be accessible by the external processor when running the software application. Data from the camera is also accessible. A camera 32 of the UVC device may be accessible by the external processor when running the software application. Data from the camera is also accessible.

The external processor, using the instructions from the software applications, may be capable of determining distance of the UVC source from the person's surroundings using the camera(s). This distance data may be used, along with elapsed time for example, to determine effective disinfection/sterilization of a targeted surface or area. The camera may also be used as part of a safety feature to inhibit UVC radiation from being directed to a person's face, including eyes. Facial recognition and calculated distance data can be used to prevent this exposure. Object/surface recognition software can also be used to inhibit exposing other objects or surfaces to UVC.

In one embodiment of a safety features, a processor, by executing instructions in a software application for example, uses the camera to inhibit harming person, animals and other objects with UVC radiation. By sizing the treatment area to match the size of the camera the live image of the camera can be used to monitor objects in the treatment area. Convolutional neural networks are known in the field of machine learning and often used in image recognition algorithms. By leveraging CNN and other known techniques in machine learning a cell phone camera can be used monitor the treatment area in real time. In one embodiment a confidence interval will be returned which will indicate the probability that an item is in the field of view or treatment area that is unsafe to treat. The system will be trained to recognize anything that can be damaged by UVC such as, but not limited to, eyes, faces, hands, cats, dogs, etc. When the user initiates a disinfection session, the UVC will not be activated until the confidence interval the treatment area is safe is at an appropriate level. When the area is determined to be safe the UVC will be activated, during the treatment time the area will continue to be monitored and if the confidence interval exceeds the predetermined threshold, the UVC will be disabled.

Existing published data on the required energy density to achieve the desired reduction in viable colony forming units has been compiled and sorted in to a quick clean setting and a deep clean setting. In one embodiment the pathogens are sorted by the required energy density required for disinfection, but it the pathogens could be grouped according to the prevalence, virulence, or other attributes. It is also considered that there could be other treatment modes besides quick clean and deep clean based on pathogen attributes and grouping. In one embodiment, an estimated log reduction can be calculated based on treatment time and distance from the light source.

In addition to using machine learning to give a confidence interval of safety, machine learning can be used to determine the distance from the UVC source to the treatment surface. Existing APIs for machine learning can be used for these calculations. The system may also be configured to determine safe use of UVC near living organisms other than the pathogen to be killed or inactivated.

In addition to calculating distance and the confidence interval of safety, the camera data can be used to determine the amount of time the UVC is pointed at a particular location. This could be with machine learning and motion detection, but also through comparing frames through subtraction for determining the amount of movement. Using these or other techniques for detection, the time at a treatment location can be determined.

Using the cleaning mode, distance, time at a treatment location (movement), and the compiled estimated treatment time equations, the user can be giving real-time feedback on the disinfection progress. The time could be an average over the total treatment time based on a movement threshold, or a more complex system could be used to track and display the treatment of objects inside the treatment area and a color map of the estimated reduction overlaid on the image.

The above teachings of the UVC device in communication with a controller e.g., processor, may be incorporated in or used with other electronic device, such as smart phone, watches and/or tablets, camera, robots, drones, vehicles, or other devices. Exemplary embodiments are disclosed below. In addition, the system may include combinations of these devices, each may include a UVC device. For example, a drone (i.e., flying robot) and a ground robot. The drone may be capable of disinfecting/sterilizing 3D space and elevated surfaces using the UVC device, and the ground robot may be cable of disinfecting/sterilizing ground surfaces using the UVC device. Also for example, motorized embodiments (e.g., robot, drones, etc.) could use an internal navigation algorithms for mapping the path traveled, these systems could be based on camera and image recognition or distance traveled. External navigation information such as GPS position, external sensors or indicators can be used in another embodiment. It is also considered the system my use both internal and external inputs for navigation.

With respect to medical application for use within the body, such embodiments can use medical navigation systems, such has BrainLab lab or other systems known in the art to treat desired tissue or organ, such as the lungs, with UVC radiation. In this embodiment, the processor may be configured to emit a UVC radiation dosage that is not harmful or not likely to cause damage to the tissue or cells of the host organism (e.g., patient) while still inactivating or killing targeted pathogen (e.g., virus) at a desired log reduction (e.g., 1-log, 2-log, 3-log, etc.) within the host organism. Other embodiments could use data from MRI, PET, CAT scans, ultrasound or other imaging information. The teachings in US Serial No. 16/113,666 are incorporated herein and are suitable for navigation.

UVC treatment can be used in combination with chemical (e.g., pharmaceutical) treatments. It could use lower 220 nanometer wavelength light. In one example, UVC can be used with hydroxy-H202 or hydrogen peroxide, 60% or greater alcohol, betadine, chlorhexidine, etc. for medical sterilization or other sterilization. This combination of treatment can be used inside the body with an endoscope, bronchoscope, fiber optic device that controls depth of penetration through lenses or power source. This combination of treatment can kill cancer cells, keloid, rapidly growing cells, tumors, hypertrophic tissue. It may also be used for sterilization as previously discussed.

UVC time for sterilization/disinfection may be shorter if there is reduced ambient light, especially sunlight. Accordingly, a light sensor may be used and analyzed to determine the amount of UVC radiation necessary for sterilization or other treatment.

It is believed UVC radiation perpendicular to surfaces is most effective for sterilization. Angles at which radiation is directed can be detected and/or controlled and different UVC devices can be configured to work together to obtain treatment of multiple or all surfaces required. In one embodiment, two devices that are providing light as perpendicular as possible to the surface one is trying to treat and one is trying to get depth with penetration. Again, this is where the optics, angles, lenses, depth of penetration, controlling the power, etc. through sensors, photo cells, photo resistors, photo diode, or photo transistors. One could use spectrometers where one could control UVC doses. The most efficient UVC is one that is close to perpendicular to the surface as possible. Whether it is in the body or whether one is disinfecting surfaces, one wants to be able to cover the surfaces with specific angles and distances from them. To control UVC application to surfaces, additional technology, such as sensors cameras or other devices are used to determine that surfaces were actually treated appropriately with the right angle and right distance. In other words, if UVC emission is not perpendicular to the surface, it may take a higher dose or longer treatment than if the UVC emission is perpendicular to the surface. Thus, the angle or direction of the UVC can be changed. Moreover, two or multiple devices can work in tandem something close, something far away, something from different angles. This is where, for example, a drone and a surface mounted robot working together may have a better approach, but these are linked together controlling doses, angles, and distances. Both multiple systems are functioning as a single unit.

In one example, vibratory energy may be applied to objects or surfaces, or the objects or surfaces may be moved by a device, such as a robotic system that moves the object to enable access to specific surface and allow change in the surface areas. The robot may not be the UVC, but the robot may be the device that moves the objects, such as food or salad in a specific location so it can be treated by UVC and effectively sterilized.

UVC Device Couplable to Hand-Held Device

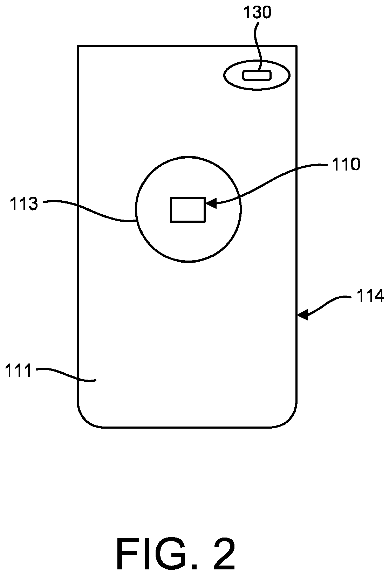

Referring to FIGS. 2, one example of the UVC device 110 is couplable to a hand-held device 114. The teachings set forth above for the UVC with Communication with Processor apply to this and other sections of the disclosure. The UVC device may be secured to the hand-held device by adhesive or in other ways. The UVC device may be part of a mobile smart phone or tablet case or secured thereto. The UVC device may be part or integrally formed with another device attachable to the phone/tablet or phone/tablet case 111, such as a grip and/or stand device 113 (e.g., a POPSOCKET device sold by PopSockets) configured to facilitate holding the phone in the user's hand or propping the phone on a surface.

Other ways of attaching the UVC device to the hand-held device are possible. For example, a magnetic coupling may be used to couple the UVC device to the hand-held device. In one embodiment the disinfection device would have an array of magnets for allowing easy mounting and removal to a phone or companion device. In another embodiment the magnetic in the UVC device can couple to a metal plate instead oppositely polled magnetics.

In an example, a magnetic coupling may be used to couple the UVC device to the hand-held device. In one example, the UVC device may be attachable to a MagSafe coupling on an iPhone 12. In this or another embodiment, the UVC device may include at least one internal sensor such as a gyroscope or accelerometer to determine the position of the device. This information would then be compared to the position of the mobile device, for example, which would be accessed via the mobile device API. When the two devices follow the same position motion patterns the processor may determine UVC device is properly connected to the mobile or other device. If the devices do not track the same motion pattern the software will determine that they are not properly connected. In one embodiment the software could prompt the user to rotate the device to the correct position if it is determined that it device connected but not in the same orientation. In another embodiment, the system might configure the device for a different mode when the device is not connected. This could disable the device, or change the user interface so that the device can be used remotely. Although this disclosure relates to the UVC device having the position sensor (e.g., gyroscope or accelerometer) and its data is communicated to the mobile or other device, other devices other than the UVC device may include the position sensor and used with the mobile or other device to determine whether the device is properly coupled to the mobile device.

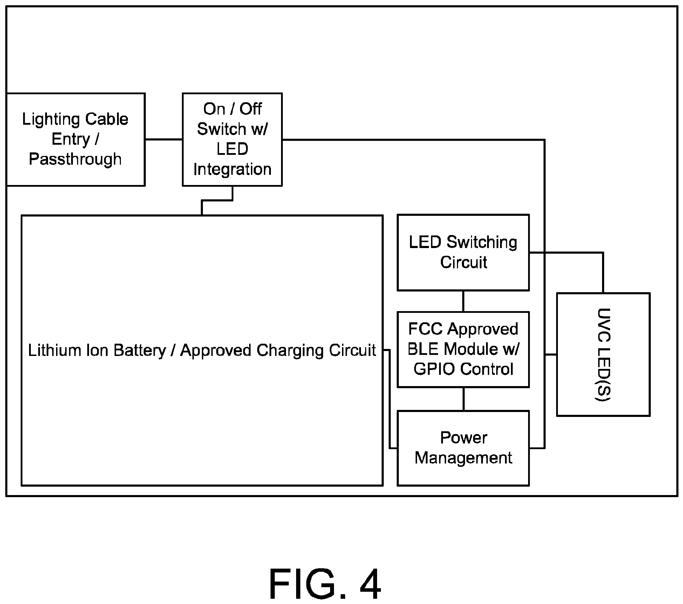

The UVC device is used by a person to disinfect an object or surface by the user holding the hand-held device and controlling the UVC device using the hand-held device, such as through a software application. Exemplary hardware of the UVC device is illustrated in FIG. 4. The UVC device is in communication (wired or wireless) with the hand-held device (e.g., a processor of the hand-held device), as explained above. As an example, the UVC device and the mobile device may be in communication via Bluetooth or other wireless communication suitable for two-way or one-way communication. The hand-held device includes camera 130.

A software application may be saved to the hand-held device and accessible by the processor of the hand-held device. The hand-held device may operate the UVC device to disinfect/sterilize a surface or object, for example, or air in the environment of the person. The hand-held device may access image date from the camera as described above to facilitate disinfecting/sterilizing of a surface or object. As an example, the software application may be used to analyze probability of 90% (log 1), 99% (log 2), or a 99% (log 3) reduction in bacteria, viruses, etc. Additional functions may be performed by the hand-held device, including detection described in the Detection disclosure section. Machine learning (e.g., AI) may be used to enhance sterilization and other functions. For example, AI may be used to determine dosages and/or wavelength for types of pathogen one is trying to protect/treat. This could be used also with wearables to determine where individuals are and if they are being treated also for social distancing with specific devices so that they can link together even if they are not part of the same package. It can detect where one wearable or one phone is where one watches. One could also use this to determine where individuals are relative to devices. This could be used for shoes, floors, surfaces so UVC on low-lying surfaces and control the log reduction where there is 1, 2, 3, up to log 6. It could be used in laboratories as well.

The targeted window or area of sterilization is generally a 12" x 12" area, which may match the camera aperture of smart phone or tablet.

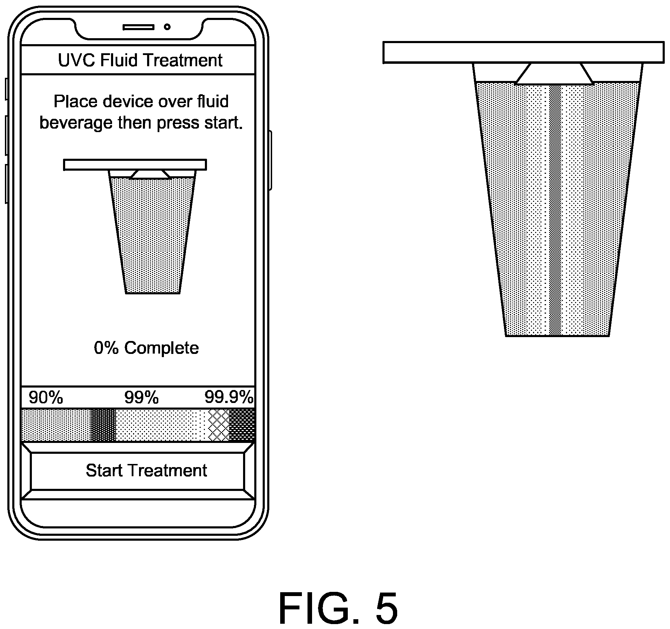

In one example shown in FIG. 5, the UVC device may be capable of sterilizing liquid. The UVC integrated into the pop socket design, for example, would be waterproof to allow for the UVC LED to be submerged into a fluid (example: glass of water). The treatment would be activated and an algorithm specific to fluid disinfection would eliminate the risk for bacteria, virus, mold & protozoan that could be present in the water. Another embodiment for treating fluids would allow the placement of the device over a cup or serving container.

This embodiment may also allow you to set the treatment time so the user could walk away, safe from UVC rays. A timer delay would allow the user to get a safe distance from the UVC before treatment starts. Notifications could be sent to a device coupled to the smartphone (example: smartwatch) allowing the user to know when treatment is over and it is safe for the user to come back into the room.

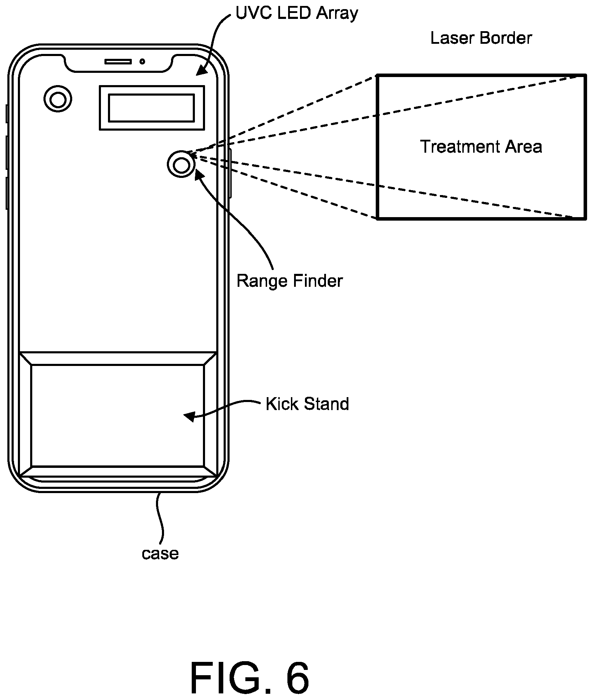

In an exemplary embodiment illustrated in FIG. 6, a case (or other item) is couplable with the smartphone (or other device, such as a smart watch or smart tablet). The case (or other item) includes one or more of a UVC source (e.g., a UVC LED array), a range finder sensor, a wireless charging battery for the UVC source, and kickstand for hands free treatment. The UVC LED array would comprise of several LEDS arranged to maximize treatment dosage. These LEDS may use a focal lens or mirror reflection. The range finder sensor could use ultrasound or laser sensing technologies to determine the range of the treatment surface. The distance between treatment surface and the LEDs will directly affect the recommended treatment time, as shown in the following equations:

$$D = \frac{P * T}{2 * \pi * L * r}$$

$$D = \frac{P * T}{2 * \pi * L * r}$$

Where:

$$D = Dosage\ Intensity\ \left(\frac{J}{cm2}\right)$$

*D = Dosage Intensity* $\left(\frac{J}{cm2}\right)$
*P = Radiant Flux (mW)*
*L = Length of LED*
*r = Distance from LED*
*T = treatment time*

*Where:*

*D = Dosage Intensity* $\left(\frac{J}{cm2}\right)$ *(^2)*
*P = Radiant Flux (mW)*
*L = Length of LED*
*r = Distance from LED*
*T = treatment time*

Solving for treatment time:

$$T = \frac{2 * \pi * L * r * D}{P}$$

The range finder may have a laser light incorporated to help with defining the treatment location. The laser would outline the treatment location with a border allowing the user to know the specific area that is treated.

The smartphone processor will define the treatment times based on treatment surface distance and predefined bacterium, protozoa and virus the treatment is intended to reduce.

The embodiment could include a kickstand to allow the phone to be freestanding and hands free. The kickstand could also be magnetic to allow for securing the phone on a metal object. The kickstand could have a clipping option so the embodiment could be clipped onto another object.

The communication between the smartphone and embodiment could also incorporate another wireless device similar to a smartwatch. The smartwatch would receive notifications on treatment times and completion of treatment, allowing for remote monitoring.

The wireless charger can be used to charge the battery powering the LEDs as well as charging the connected smartphone.

In another embodiment the light source will operate at multiple wavelengths, allowing for visible light, UVA, UVB, and UVC options. The visible light would work as a flashlight.

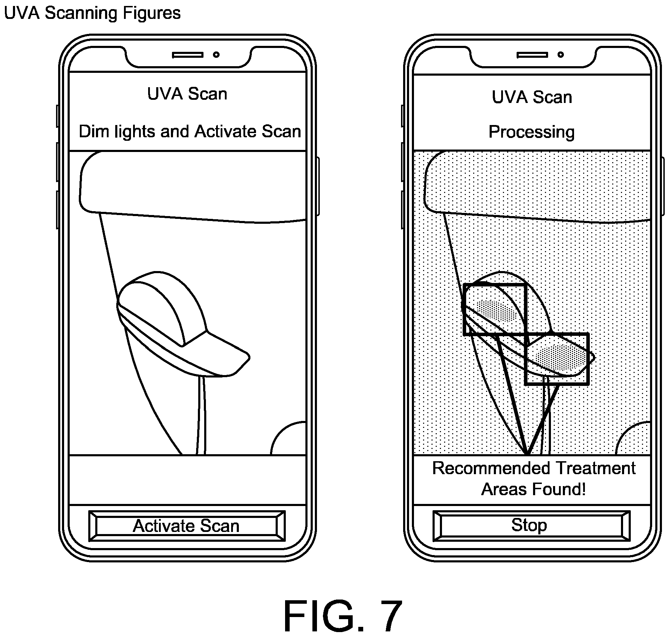

In addition to the UVC wavelengths the LEDs could operate in the UVA range. When operating in the UVA range the camera could be used to detect possible areas requiring disinfecting, as shown in FIG. 7.

In one embodiment, incorporating magnetic manipulation into a UVC system would allow for wavelength shifting in a continuous or pulsed pattern. For example, continuously pulsing is a specific pattern to enhancing the sterilization efficacy. The magnetic manipulation may also be used as a safety feature to discontinue lower UVC intensity when the UVC device is near a low magnetic force. This may be applicable for robotic cleaning systems remotely cleaning. Magnetic emitters could be placed near the patient or UVC sensitive product and when the robotic cleaner gets near the magnetic emitter the LEDs would lower or completely stop within the magnetic field.

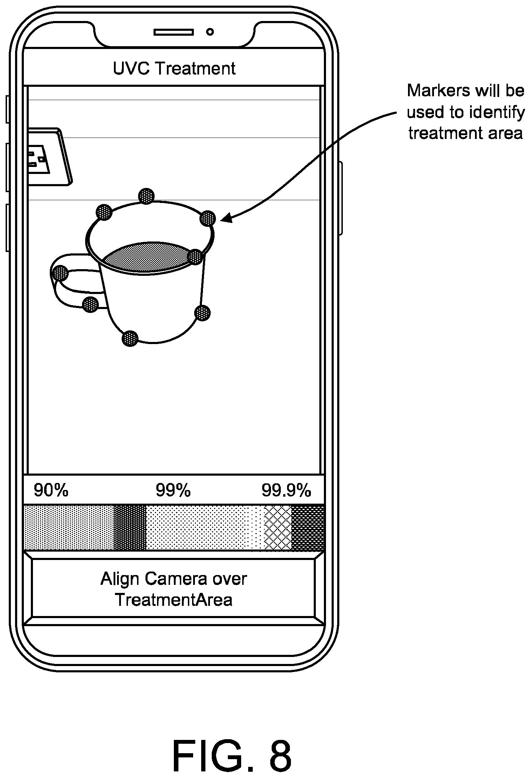

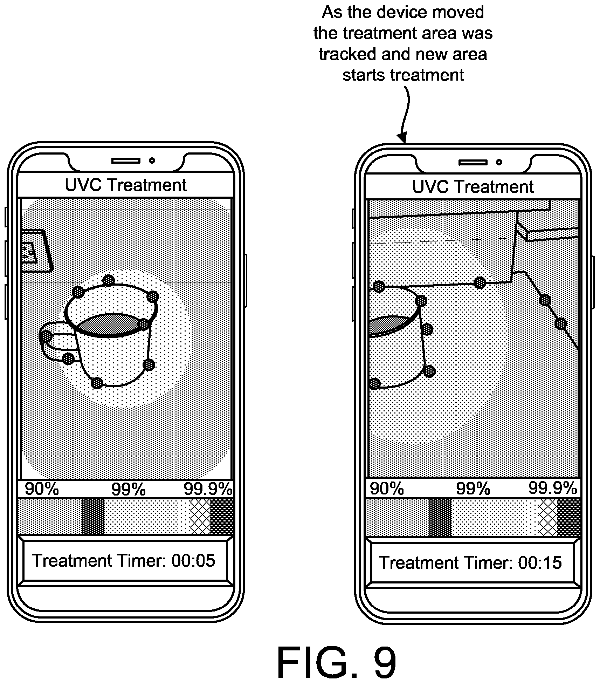

As shown in FIGS. 8 and 9, during treatment the camera will be directed towards the treatment area. The longer the UVC is treating that area the screen will display a filter over the displayed image changing color based on treatment dosage for that location. As the user moves the device over a different area the filter identify markers and determine if that area has been treated and adjust the filters accordingly. The dosage will be based off of time, distance and known UVC intensity. Distance can be determined using a smartphone distance API or a hardware proximity sensor. Alerts and notifications can indicate to the user when to move the device based on desired disinfectant requirements. If the user prematurely ends treatment before desired disinfectant, then the device will alarm the user.

It is also considered that the system could use advanced imaging technologies, such as LIDAR, to create 3D mesh or grid on the objects being treated. This would allow a more precise calculation of exposure to each area in the grid. The treatment amount in each grid could be represented by a color overly, so the use would give a visual representation of the treatment similar to a heat map.

## Layered Face Mask

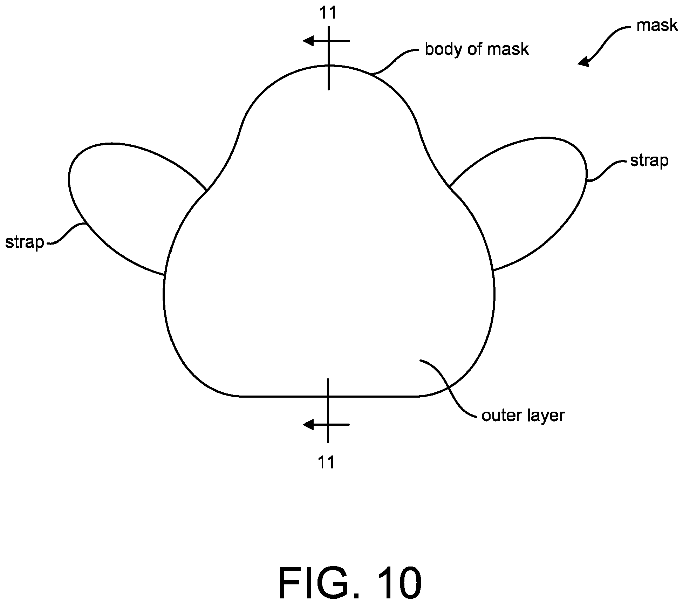

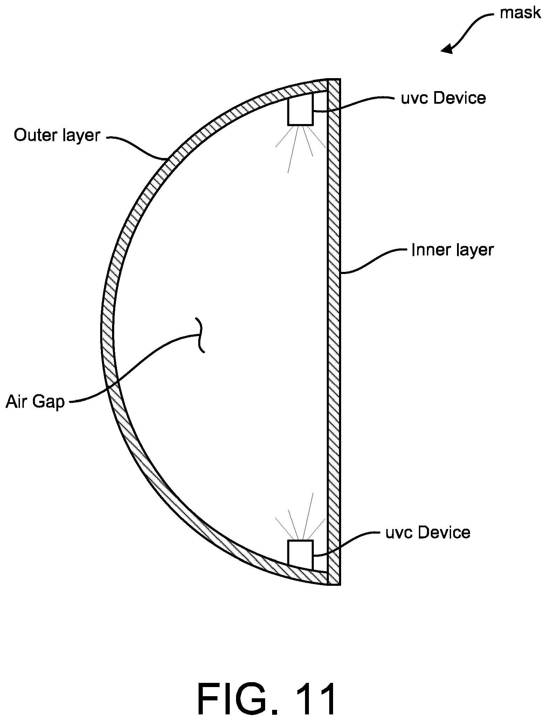

One example of a face mask using UVC is indicated generally by reference numeral 10 in FIGS. 10 and 11. In this example, the face mask includes opposing inner and outer layers, and a UVC device (including a UVC source) between the inner and outer layers. One or both of the layers may be capable of filtering pathogens. Alternatively, the layers may not be suitable for filtering pathogens. The inner and outer layers may be flexible or semi-rigid. The inner and outer layers may be woven or non-woven. The inner and outer layers may include natural or synthetic fibers. In one example, the outer layer may have a higher filter rating than the inner layer, which may not be a rated filter. For example, the outer layer may be an P95 filter (rated to remove 95% of all particles that are at least 0.3 microns in diameter), or an P99 filter (rated to remove 99% of all particles that are at least 0.3 microns in diameter), or an P100 filter (rated to remove 100% of all particles that are at least 0.3 microns in diameter). The inner layer may not be rated to filter particles that are 0.3 microns. The inner layer may be lightweight and pliable.

The inner and outer layers define a space or air gap there between. The UVC device directs the UVC radiation in this air space. When donned on the person, air flows through the outer layer into the air space and then through the inner layer to the respiratory system of the person. As the air flows in the air space toward the inner layer, the UVC radiation from the UVC device irradiates the particulate in the air, including any pathogens traveling on droplets of fluid such as from an infected person to decrease or substantially eliminate pathogen load (e.g., viral load) entering the respiratory tract of the person. In one example, the outer layer and/or the inner layer may be disposable and replaceable to inhibit cross-contamination. In another example, the inner and/or outer layers may be individually sterilized by UVC and/or chemical treatments or other treatments so these are not constantly disposed of as millions of these masks are produced on a daily basis and are required. Thus, the inner layer and/or the outer layer may be suitable for being sterilized after use. A UVC shield may be provided to inhibit or reduce the amount of UVC radiation emitting outside of the face mask. The inner layer and/or the outer layer may be suitable to inhibit or reduce this emission or another layer of structure may be integrated therein.

As with the other embodiments, the UVC device may be controlled by a processor (e.g., a smart phone or tablet) accessible by the person. Alternatively, the UVC device may be powered on the person and constantly emitting UVC radiation until the person turns the device off. In this example, the UVC may not be controlled by an external processor.

The UVC could project from the front of an individual so as one is breathing air this could be sterilized before one is even breathing so that if the light or UVC is faced forward and control the direction with or without a mask, filter, or screen. This could essentially project forward to protect the air one is breathing either in combination with a mask or other protective device. It could also be used to sterilize personal protective equipment.

## Face Mask with Sterilizing Plenum

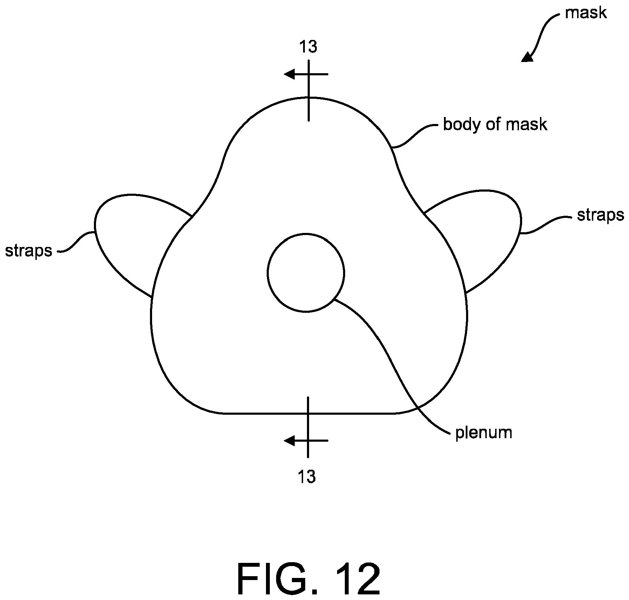

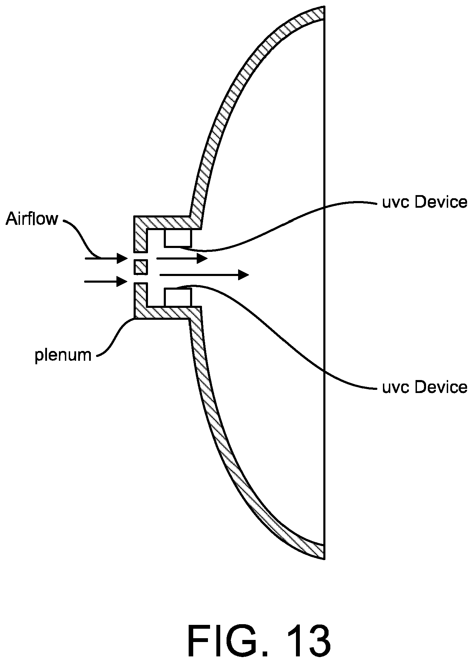

One example of a face mask or shield using UVC is indicated generally by reference numeral 10 in FIGS. 12 and 13. In this example, the face mask includes a mask body that is substantially air impermeable and blocks air flow containing pathogens, and a sterilizing plenum coupled to the mask body and configured to supply the person with ambient/environmental air. The sterilizing plenum includes a UVC device suitable to irradiate and sterilize air flowing through the plenum and to the person.

The UVC device may include one or more UVC LEDs (e.g., a plurality of UVC LEDs) or alternative sources of UV. As with the other embodiments, the UVC device may be controllable by a processor, such as a smart phone or tablet or may be continuously active and turned off and on by the person. Filter medium may be in the plenum or over the mouth and nose of the person.

In one or more examples, the UVC device (or separate device) may include one or more sensors in communication with a processor (e.g., external processor, such as smart phone or tablet). The sensors are configured to detect a parameter of the air that may enter the plenum, that is within the plenum, and/or that exited the plenum. It is understood that these sensor(s), and those described below, may be incorporated in the layered face mask embodiment described above to determine a parameter of the air that may enter the air space, that is within the air space, and/or that exited the air space. The processor is configured to perform a function based on the data received from the sensor(s).

In one or more examples, the UVC device (or separate device) may include an airflow sensor in communication with a processor (e.g., external processor, such as smart phone or tablet). The airflow sensor is configured to determine the airflow rate through the plenum, and may be within the plenum or otherwise in communication therewith. This airflow rate may be use by the processor to determine the person's breathing rate. In one example, the processor may control the amount of UVC radiation being emitted based on the breathing rate. If the person is for example breathing very heavily and there is a high flow rate, then the processor may activate additional UVC LEDs (or other sources) for example to increase irradiation. Thus, the amount of UVC radiation being emitted may be controlled by the processor based on the airflow rate. The processor may also communicate with the person, either auditory, visual, or otherwise, the amount of sterilization being performed. For example, a warning indicated may be communicated if the breathing is too rapid and the UVC device is unable to sterilize at an acceptable rate. The estimated sterilization may be also be communicated and indicated as to whether the user may be at risk for exposure to pathogens.

In one or more examples, the UVC device (or separate device) may include an image sensor (e.g., camera) in communication with a processor (e.g., external processor, such as smart phone or tablet). Data from the image sensor may be used by the processor to determine if particulate of certain sizes may be entering the plenum, are within the plenum, and/or are exiting the plenum and potentially entering the respiratory tract. This information may be communicated to the person by the processor. This information may be used by the processor to increase irradiation or perform other functions.

Other Wearable UVC Devices

The UVC device may be otherwise provided to be removably worn by a person and configured to disinfect/-sterilize air being inhaled by the person (and even exhaled by the person to protect others). For example, the UVC device may be integrated or otherwise attachable (removably or non-removably) to a watch (e.g., a smart watch), hat, a headband, a strap, a garment, headway, face shield, glasses, or other wearable item. When being worn by the person, the UVC device emits UVC radiation to the air adjacent the person's face to irradiate and sterilize the air that is most likely to be inhaled (and exhaled) by the person. The UVC device may include a plurality of UVC sources (e.g., a plurality of LEDs) capable of emitting UVC radiation at least partially around the person, such as 90 degrees, or 180 degrees, or 270 degrees, or 360 degrees. For example, the LEDs may be provided on a flexible circuit board.

As with the other embodiments, the UVC device may be controllable by a processor, such as a smart phone or tablet or may be continuously active and turned off and on by the person. In one or more examples, the UVC device (or separate device) may include one or more sensors in communication with a processor (e.g., external processor, such as smart phone or tablet). The sensor(s) are configured to detect a parameter of the person's surroundings and communicate with the processor. The processor is configured to perform a function based on the data received from the sensor(s).

In one or more examples, the UVC device (or separate device) may include an image sensor (e.g., camera) in communication with a processor (e.g., external processor, such as smart phone or tablet or watch). The image sensor be used as part of a safety feature to inhibit UVC radiation from being directed to another person, including eyes and skin. Software using image data from the image sensor for object recognition (e.g., facial or human recognition) and calculating distance can be used to prevent this exposure. Object/surface recognition software can also be used to inhibit exposing other objects or surfaces to UVC. In one example, the software application may instruct the processor to turn off the UVC source (e.g., one or more UVC LEDs) when a person or other object is detected within a certain distance. A warning (e.g., audio, visual, tactile) may be communicated with to the person indicating that the UVC source is turned off. A warning (e.g., audio, visual, tactile) may be communicated to the other person. A communication may be sent also when the UVC is operating. Other embodiments could include thermal imaging sensors.

For example, the power level and environment for safety may be monitored. In one embodiment the object being treated will be looked at to determine if the material of the object being treated. Many polymers can be damaged by prolong exposure to UVC, and to prevent this damage or discoloring of the polymers and other materials. In one embodiment the intensity of the light source can be modulated to ensure that only the energy required to reach the desired disinfection level is used. This smart dosage calculation will ensure proper levels of reduction while minimizing the damage to materials. Rather than random high energy to provide sufficient energy at furthest locations and over treat more proximal or near surfaces - calculations and optimization to give the surface appropriate dose and move device closer or modulate energy by location direction intensity or multiple devices working in unison to treat surface, tissue , air fluid etc. This could be by moving device direction or proximity to object or for example have floor mounted and drone mounted device to give appropriate energy, or could be augmented with chemical or pharmaceutical means to optimize. Not just human DNA or lenses are damaged by excess doses but polymers are damaged - weakened discolored, paint affected, bonding surfaces like grouting etc. This equipment or surfaces can be expensive to replace or resurface color etc. and so optimizing surface energy important.

As another safety feature, the UVC device or a separate device may include a visible light source or other warning, including audible, communication to others around the person that the UVC device is operating. This may warn others to stay at a safe distance from the person and also facilitates social distancing.

As yet another safety features, optics (e.g., lens) may be used such that the emitted UVC is less likely to be harmful to persons, animals, or objects beyond a certain distance, such as two feet or other distances. In addition, there may be timers built in to your mobile device, watch, or other mobile system that would alert you that the individual near you might be receiving excessive UVC radiation.

In one exemplary application, the wearable UVC device may be employed in a hospital setting, such as an emergency room or in an ICU, or other designated areas. In such a use, persons in the area may don the UVC device, such as explained above, and wear protective eye coverings that filter out the UVC radiation (e.g., glasses) emitted from others wearing the UVC devices. Suitable clothing may also be worn to prevent skin damage from the UVC devices.

## 2. Infection Risk Detection

The following infection risk detection disclosure may be combined with any of the UVC disinfection/sterilization embodiments or may be used independently thereof. In general, an infection risk detection system includes at least one sensor configured to detect at least one environmental parameter around a person, and a software application executable by a processor for determining an infection risk of a person based on the detected environmental parameter(s).

Examples of environmental parameter sensors are described below. The system may include one or more of the sensors. The sensors may be configured to be worn by a person or are otherwise mobile and can be carried by the person, whether in the person's hand or in a person's pocket. The sensors may be integrated or otherwise attachable (removably or non-removably) to a hat, a headband, a strap, a garment, headway, or other wearable item. The sensor(s) may be incorporated in an electronic device, such as a smart phone, smart watch, smart tablet, wearable camera, and/or other devices. When being worn or otherwise carried by the person, the sensor(s) detect an environmental parameter to be used by the processor, to which the sensor(s) is in communication, to determine a risk of infection by a pathogen. The processor may be incorporated in the sensor(s), or within the same housing as the sensor(s), or external to the sensor(s).

Light Sensor(s)

In one example, the sensor may be a light sensor. The light sensor may be capable of detecting visible light or non-visible radiation, such as infrared radiation.

With respect to a light sensor detecting visible light, the light data from the sensor may be used by the processor to detect particles in the environmental air. When running a software application, on a smart phone, smart tablet, or smart watch, for example, the processor may process and analyze the light data from the light sensor to determine particulate size in the environmental air. If the particulate size is commensurate with possible pathogens, the processor may communicate a warning (visible, audible, and/or tactile) to the person.

Also with respect to a light sensor detecting visible light, the light data from the sensor may be used by the processor to detect distance from other persons. When running a software application, on a smart phone, smart tablet, or smart watch, for example, the processor may process and analyze the light data from the light sensor to determine if another person is within a predetermined distance of the person and not practicing social distancing. The processor may communicate a warning (visible, audible, and/or tactile) to the person.

With respect to a light sensor detecting infrared light, the light data from the sensor may be used by the processor to detect the temperature of another person in proximity to the person. When running a software application, on a smart phone, smart tablet, or smart watch, for example, the processor may process and analyze the light data from the light sensor to determine whether the other person may have an elevated body temperature, indicative of a fever. If this is the case, the processor may communicate a warning (visible, audible, and/or tactile) to the person.

Auditory Sensor(s)

In one example, the sensor may be an auditory sensor. The auditory sensor may be capable of detecting sound. The auditory data from the sensor may be used by the processor to detect whether another person in proximity is making respiratory sounds that are indicative of a disease caused by a respiratory infection. Such sounds may be labored breathing, wheezing, or other sounds. In other examples the acoustic sensors could be include an emitter and a receiver. The emitted sound could be in the audible range as well as ultrasound and/or infrasound.

Chemical Sensor(s)

In one example, the sensor may be a chemical sensor. The chemical sensor may be capable of detecting chemicals expelled from a person's respiratory system that is indicative of a respiratory infection. The chemical data from the sensor may be used by the processor to detect whether another person in proximity is exhaling one or more chemicals that are indicative of a respiratory infection. Such chemicals may be pyruvate, ammonia, ketobutyrate, hydrogen sulfide, dimethyl sulfide, nitrates, mercaptan, or other chemicals.

Biosensor(s)

In one example, the sensor may be a biosensor. The biosensor may be capable of detecting one or more pathogen expelled from a person's respiratory system that is indicative of a respiratory infection. The biosensor may comprise a miniaturized analytical device integrating biological probes such as enzymes, antibodies, nucleic acids, peptides, viruses, etc., with a transduction platform able to pick up attachment events between the probe and the target. The transduction platform may include one or more types of physical phenomena including optical, mechanical, piezoelectric, amperometric, etc. The data from the sensor may be used by the processor to detect whether another person in proximity is exhaling one or more pathogens that are indicative of a respiratory infection and/or the presence of pathogens in the environmental air around the person.

Additional

In addition to using both internal and external sensors to determine an infection risk, additional information can be obtained from crowdsourced data. For example, using known information about popular times, wait times, and visit duration available through Google Maps or other API, the software could alert the user when they are entering an area which currently has a large amount of people or has recently had a large amount of people. This alerts could help inform the user to be more vigilant about using disinfection methods based on the increased exposure.

In another embodiment the proximity of other people could be done via scanning of common wireless protocols. For example, the system could scan Bluetooth and/or WiFi and check the amount of advertising devices to estimate the proximity of people. This technique could be used separately or in conjunction with other methods described.

Another embodiment would upload the usage data of the system the cloud along with GPS locations of where the device was used. This information could be anonymously shared when the user was in the same area, so they were aware the last time it was disinfected by another system.

### 3. Medical Device Disinfection/Sterilization

The following medical device disinfection/sterilization disclosure may be combined with any of the UVC disinfection/sterilization embodiments and/or infection risk detection embodiments, or may be used independently thereof.

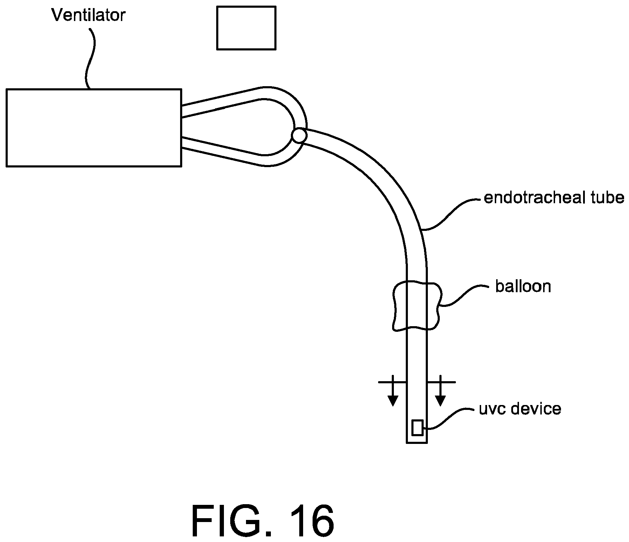

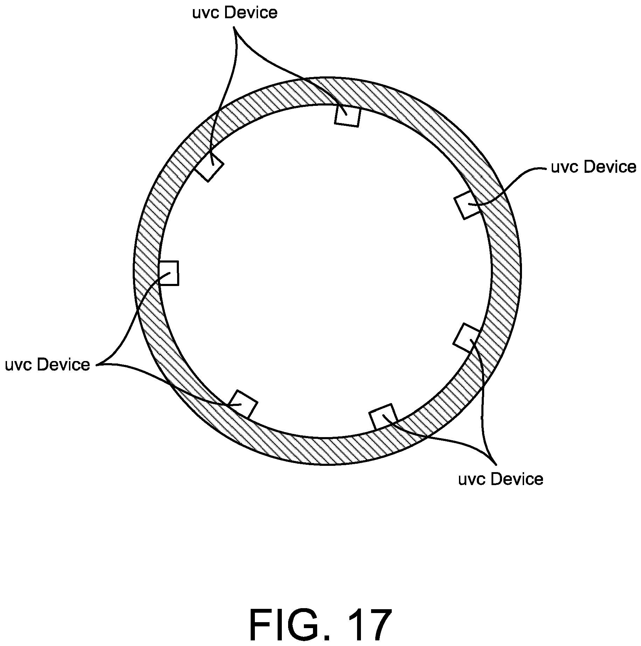

Referring to FIGS. 16 and 17, in general a sterilizer is configured to perform sterilization inside a catheter, cannula, tubing, or other medical device defining an internal passage for the delivery of fluid, gas, a medical device, or other deliverables (broadly, a medical delivery device) which is insertable into a patient's body. The sterilizer is capable of sterilizing the delivered fluid, gas, and/or medical device, etc., inside the patient's body and within the medical delivery device. In the illustrated example, the sterilizer is coupled to the medical delivery device and is insertable into the patient's body with the medical delivery device. In one example, the sterilizer is received in the internal passage of the medical delivery device and is capable of sterilizing the delivered fluid, gas, and/or medical device, etc. as it passes therethrough. The sterilizer may be disposed adjacent the distal end of the medical delivery device so that sterilization takes place generally immediately before entry into the patient. This is believed to give the best probability of maintaining sterilization before entry into the patient and reducing contamination/infection. The UVC device may be combined with other disinfection/sterilization devices and methods or be the sole source of disinfection/sterilization. For example, the UVC made be combined with one or more of: filter media for filtering pathogens (e.g., a respirator or face mask), chemical disinfectants (e.g., alcohols, hydrogen peroxide, hydrogen peroxide vapor (HPV), among others), which may be received in the medical delivery device.

In the illustrated embodiment, the medical delivery device is an endotracheal tube placed into the trachea to provide air/oxygen to the patient, such as by a ventilator. It is understood that the teachings of this embodiment apply equally to each of the other medical delivery devices. The illustrated sterilizer is a UVC source, such as one or more UVC LEDs, although the sterilizer may be of other types. UVC shielding is provided, such as being coupled to or incorporated in the body of the endotracheal tube to inhibit UVC radiation from entering the patient's body. For example, the tube itself may be opaque to UVC and may have UVC absorbing polymers or colors for shielding local tissue inside the patient's body. The UVC LEDs may be provided in an array extending around the interior of the tube, such as 360 degrees around the interior, and emit light toward a center axis of the tube.

With ventilator systems, the air that is coming is filtered upstream of the endotracheal tube. Typically, ventilators for example filter the air going into the ventilator from the room, but the patient's inspiration and expiration with air back and forth where patients are seeding themselves. With the present endotracheal tube with the UVC device, as the patient is breathing in and out, the air is continuously sterilized in the tube rather than the patient continuing to receive the viral load which increases the damage. The UVC device disinfects/sterilizes as deep as possible so that exhaled air is sterilized.

In another embodiment, sterilization could be done with bronchoscopy and for example laser light and LED where one could actually sterilize very quickly the bronchi, mouth, stomach, nose, etc. For example, any space where you would project this with timers knowing the time, distance, depth, and sterilization amount so you could try to effectively maybe not 100% sterilize but reduce the bacterial viral/fungal load within the body very quickly without damaging the local tissue or shielding the local tissue as much as possible. This could be done through surgical management or treatment.

## B. Treatment

The following treatment disclosure may be combined with any of the UVC disinfection/sterilization embodiments and/or infection risk detection embodiments and/or medical device disinfection/sterilization disclosure, or may be used independently thereof.

In one or more examples, UVC may be used to treat cancer. UVC treatment from a suitable device may be used to break down DNA/RNA to treat cancerous tissues or other fast growing or hypertrophic tissue, such as a keloid.

In one or more examples, UVC may be used to sterilize an organism, such as an animal or human. UVC treatment from a suitable device may be used to sterilize, temporarily or permanently, either a male or female organism. This can be used with other agents for sterilization. This can be performed as "on demand" to sterilize or inhibit cell growth.

In one or more examples, UVC may be used to treat an infection of an organism, such as an animal or human. For example, UVC could be administered through lasers, switching through bulbs (fluorescent or LED). It could be done with pulse therapy so fraction of a second and on a repeated basis. UVC treatment can be combined with chemical treatment. This could be used, for example, to treat the pathogens in the eye like toxoplasmosis or parasites versus treating cancer cells. Optics may be used to determine the depth of penetration to the tissue. For example, where an infection is superficial like a respiratory infection (e.g., coronavirus), then optics are used to limit the depth of UVC into the tissue to only treat the infection (e.g., treat the pathogens causing the infection). The UVC can be delivered circumferentially, where the UVC radiation is emitted in multiple directions, a single direction, or in bursts. The UVC treatment can be combined with visualization of the infected area or the lesion (in other words, the tissue to be treated) and one could treat immediately or this could be done on a time or controlled basis. This provides treatment of damaged or infected tissue or pathologic tissue i.e. tumor, infection, etc. The UVC light sources also could be used potentially for diagnosis, especially if one used OCT based technology to do this.

In one exemplary method, fluorescent guided injections can be radiolabeled to have markers on antibiotics or immunoglobulins. One could then introduce an endoscope into the body or bronchoscope to see the pathologic tissue, which is then fluorescent. One could then use burst therapy with UVC to treat the damaged tissue. One could use fluorescent type guidance to therapeutic type guidance. One could use different types of lenses - fresnel lens, angle lenses, prisms, different types of optics controlled adaptive penetration. If this is in the subsurface tissue, one can use a minimally invasive approach, such as described in the application incorporated by reference above herein. One could put a balloon or dissect carefully into the tissue and then treat the tissue around it. One could also use mirrors to direct the UVC laser. Note, treatment is based on the line of sight with light and then controlling the angles at which the UVC is directed to the tissue to be treated. One could also use electrical charges or cameras that have very high magnification because of pathological treatment and then treat this pathological tissue.

UVC can be powered through a "biological battery". Battery could be developed through motion, heat, flexibility. These could power the internal or external UVC that are applied to sterilize surfaces or sterilize within the body. The systems can also create a reservoir with pharmaceuticals or chemical agents to treat in addition if they are needed at angles or directions that the direct UVC light cannot penetrate.

Infections or other illness may be detected with sensors. The infection or other illness can then be treated with UVC. Sensors can also be used such as graphine sensors or flexible sensors. The graphine sensors could have artificial sense of smell, place DNA on graphien silicone wafer that allows resistor or sensor links to the sense of smell. This type of sensor as an example could be used to detect damaged, injured, or infected tissue on a very fine level and then use this as a guide to treat for the UVC. For example, infections have certain smells to bacteria, viruses, parasites often as either rapidly growing or decaying which create these smells. These graphine silicone layered sensors can be utilized as "artificial nose". These type of sensors and other application may help.

## C. Other Uses

The following treatment disclosure may be combined with any of the UVC disinfection/sterilization embodiments and/or infection risk detection embodiments and/or medical device disinfection/sterilization disclosure, and/or treatments or may be used independently thereof.

Human-Posed Estimation (HPE) with Artificial Intelligence can be used to determine the UVC applications.

Navigation technology, magnetic or other navigation can be used to direct or locate the tissue that has been treated. This can be with or without fluorescents or other immunoglobulin or antibody bonded systems which would bind the tissue or surfaces that need to be treated and treat these. UVC can be used with robots to control navigation whether these robots, drones, others. It could be both inside and outside the body or linked to additional robotic systems.

UVC application can be used in food services. For example, UVC application can be used for salad spinners or other devices in refrigerators that protect individuals applying the appropriate dose removing or vibrating the material so one can get even dispersion over all the surface to disinfect without damaging the materials, food, etc. UVC application can be used for processed foods that are not necessarily cleaned or sterilized such as fresh seafood, meats, salads, etc. This could also be done in restaurants where lights would be applied to a surface. Rather than just heating the food products, UVC application can be used to sterilize them knowing the doses and angles. Hands could be protected by opaque gloves, for example, when one is working on this. Also, one could use gloves or motion apparatuses or even robotics to allow motion to the food so sterilization/disinfection while the food is waiting either to be processed or to be served. This may benefit every kitchen rather than just having heating lights or cooking materials. There could be filters through UVC whether everything from waters to soups to salads to disinfecting surfaces without actually damaging the food. This could be linked with or without thermal applications, chemical, pharmaceutical, etc.

UVC application can be used between two air filters or between a filter or a screen such as a face mask and a mask. Between those two, the air could be sterilized. This creates a zone that the UVC is filtering as well as the mask and/or face shield.

As another example, gloves or other personal-protection equipment can be sterilized using UVC application.

The UVC device may be fixed to appliances such as standard light fixtures. The UVC device may emit visible light and UVC radiation, such as 220 or 254 nanometers in the area that would sterilize for a period of time and disinfect. Using existing light fixtures with or without camera sensors, light power sensors, etc., one would be able to use.

One could use UVC device with a filter within a mask. One could use this with MagSafe technology with a magnet. One could use it with a pouch.

UVC application can be used in the eye, for example, in the human body for laser optics where one would kill the toxoplasmosis (an infection in the eye) with burst type therapy. It could also be used for tumors in the eye, melanoma, etc. It could be applied to chemotherapy agents, oncologic agents. These optics would work together to link to treat specific malignant tumors whether in the eye, any other tissue, etc.

HVAC systems could slow the air down while it is being treated and then speed the air up through the HVAC system with/without filters or with/without local chemical agents.

One could also create a shield with mirrors between the two and UVC can reflect off the mirrors or other reflective surface. There is some reduction in power and strength that can be used to get around to all the specific surfaces one is trying to treat. Other reflective material can be used to enhance delivery of UVC radiation to surfaces, such as surfaces of food, for reducing active pathogens, such as e. Coli.

UVC application can be used in bathrooms especially showers and around toilets. It could be used between individual patients treating around the floor and on all surfaces. UVC application be used in cars, vehicles, HVAC, respirators, trucks, anesthesia machines, integrated intratracheal tubes. It could be used with different types of camera systems to magnify or see the particles. This could be used with infection risk detection. It is described in the microclimate as well as infection treatment with PhPO2, heart rate, temperature changes, and also sensory such as smell so you can identify, localize, and treat.

## D. Visualization of UV Light

The following disclosure relates generally to the visualization of ultraviolet light. This disclosure may be used in conjunction with any of the above disclosures, but these visualization techniques are not limited to the above applications.

UVC is a known method for surface and air disinfection, but one of the limitations of the technology is that the light is not visible to humans, making it difficult to know if the surface has been properly cleaned. The disclosure relating to the use of a mobile device (e.g., phone) and augmented reality to visualize UVC treatment was made. In addition to or alternatively, a complex data structure can be created to detect UV light.

In one example, a 3D representation of the space is created. This can be done with existing software libraries such as ARCore, ARKit, or a combination of machine vision and accelerometer data. In addition, accelerometer data and other sensor data can be combined with frameworks such as ARKit and ARCore. Using the 3D representation, nodes can be created and sized based on the distance of the detected treatment surface from the light source. In the case of an LED, the light source can be thought of as a point source where the area location is calculated using the following equation:

$$A_{treatment} = \pi \left[ h * \tan \frac{\theta}{2} \right]^2,$$

where

LED Point Source

h

r

Fluence is then calculated using the following equations:

$$\frac{mJ}{cm^2} = \frac{mW * S}{A_{treatment}} = \sum \frac{mW * \Delta S}{\pi \left[ h * \tan \frac{\theta}{2} \right]^2}$$

For each node created, the distance (h) is sampled at a predetermined value and used calculate the fluence where ΔS is the sample rate. Other known sampling techniques can also be used in the calculations as well. The calculated fluence can then be stored at each node location. This information would then be relayed to the user though heat maps which could display the amount of time at each location, the fluence at each location, an estimated reduction of a pathogen at each location based on meta analysis or individual UVC dose-response relationships. This could be done in tabular notation, text overlay, or through an augmented reality heat map. Other known data visualization methods.

In another embodiment, this could be used for other application outside UVC disinfection and applied to other wavelengths. One embodiment could use ultraviolet in the wavelength 320 nm to 395 nm. This wavelength is used in UV curing applications. UV curing typically uses the effective energy density C4), which is calculated in the same method as the UVC fluence. For curing applications, a 3D map would be created of the work area including the pieces being bonded. This information could then be relayed to the user in known data visualization methods. In one embodiment, the user could select materials being joined from a lookup table with known effective energy density required for successful bonding.

This light detecting technique could be used to indicate when the user should move to a new location when using a handheld device. In another embodiment, a target bonding area could be preprogrammed in the system. Using machine vision, the system could be preprogrammed to recognize multiple preprogrammed parts. When a part is recognized based on preprogrammed instructions, an overlay of the targeted bonding area can be overlaid on the screen such that the bonding area is to be "colored in" with data from the ED effective energy density map. It is also considered that the technology could be used in a multi-wavelength system, where the energy information on each wavelength could be stored separately. The effective energy density over the entire bond area could be analyzed to come up with a prediction of the bond quality.

In addition to displaying energy dosage information in a 3-dimensional (3D) space, the information about cleaning, bonding, or stored information could then be uploaded to a local server or to the cloud with timestamps and other information. This information could then be used in process and quality control in industrial applications, or be available to uses in consumer applications for review.

These systems could use a mobile phone or tablet device, or a standalone system with the camera and light source integrated. The standalone system could have a screen to display the output to the user, or other known indicators such as LEDs could be used.

In an embodiment, the target disinfection levels may be selected by the user. It is also considered that the target levels could be pre-selected in the application. Using the selected target sanitation time, the total time can be determined for the treatment for each location.

## E. Adapter for UV Disinfectant Device

In general, the following description is directed to an adapter for an ultraviolet (UV) disinfection device to enable the disinfection device to be coupled to a container for disinfecting an interior of the container. In the illustrated embodiment, the adapter is a lid adapter configured to function as both a lid (e.g., lid, cap, top, etc.) for a hand-held, reusable fluid container (e.g., a bottle, cup, etc.) for holding drinkable fluid (e.g., water or other liquid), and an adapter for a UV disinfection device to enable the UV disinfection device to safely disinfect an interior of the fluid container.

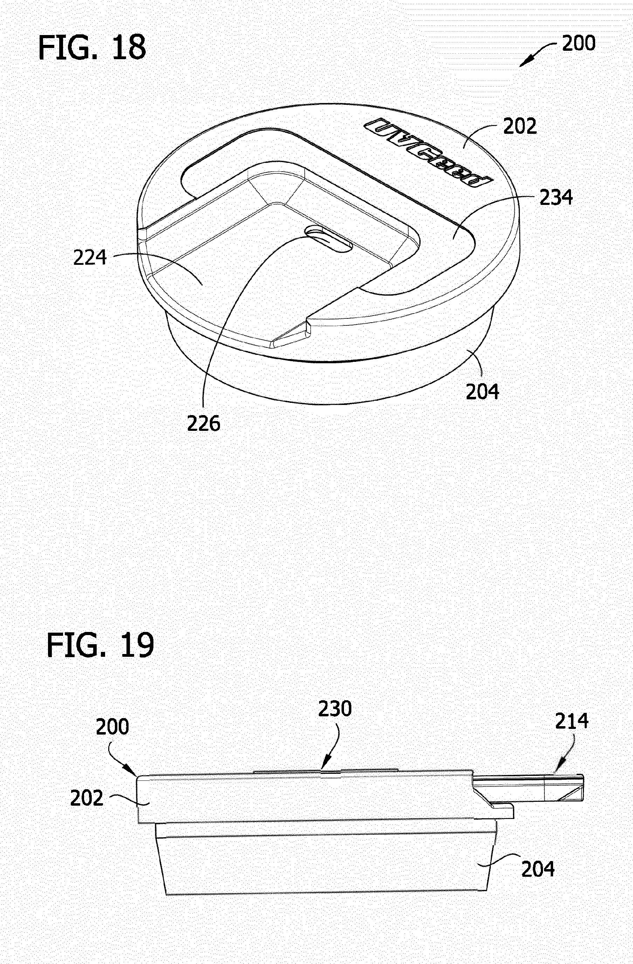

Referring to FIGS. 18, 19, and 22-24, an embodiment of lid adapter for an ultraviolet (UV) disinfection device is generally indicated at reference numeral 200. The illustrated lid adapter 200 includes an upper lid body 202 and a lower bottle coupling 204 (broadly, adapter coupler). As described below, the lid body 202 is configured to function as an adapter for the UV disinfection device to enable the UV disinfection device to safely disinfect an interior of the fluid container. The lid body 202 may be formed from a suitable plastic, such a polypropylene or copolyester, or other suitable material, including but not limited to stainless steel, aluminum, HDPE or solid silicone. In one example, the lid body 202 may be opaque or generally opaque to UV light, so that the UV light from the UV disinfection device is inhibited from traveling through the lid body.

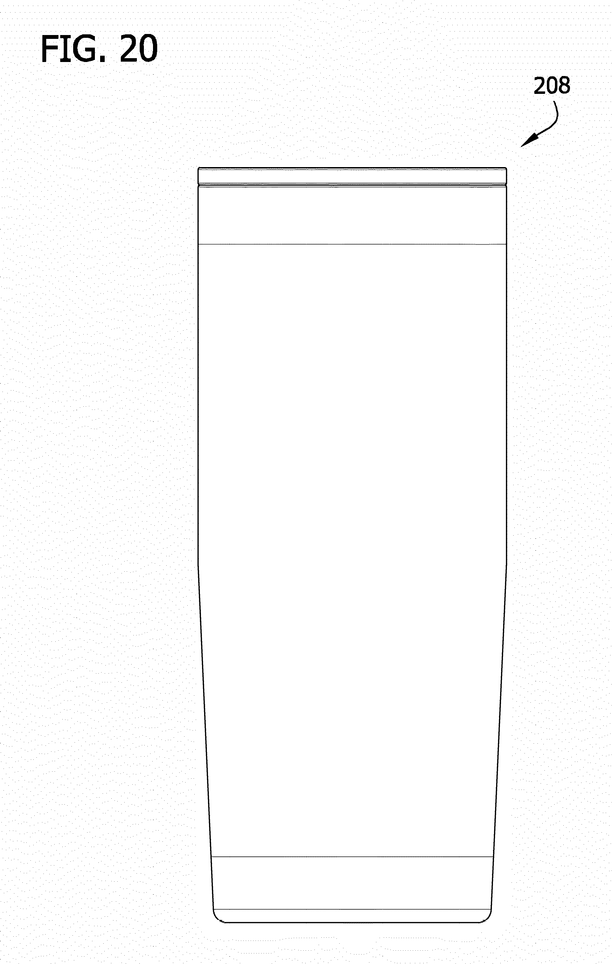

A suitable container (e.g., reusable bottle) for use with the lid adapter is shown in FIG. 20 and generally indicated at reference numeral 208. Such bottles 208 are sold by companies such as YETI®, HYDRO FLASK®, STANLEY® drinking cup, among others. The lower bottle coupling 204 of the lid adapter 200 may be elastically resilient, such as an elastomer (e.g., rubber), configured to removable couple to different bottle types from different manufactures, such as YETI® Yonder 750 mL, STANLEY® 40 Oz Tumbler, and the HYDRO FLASK® Wide Mouth Water Bottles. In other embodiments, the bottle coupling 204 may be a coupling specific or proprietary to the certain type or brand of bottle. For example, in one or more embodiments the lower bottle coupling may be a non-elastic polymer that screws into or is otherwise is couplable to the top portion of the container or one or more different types of containers.

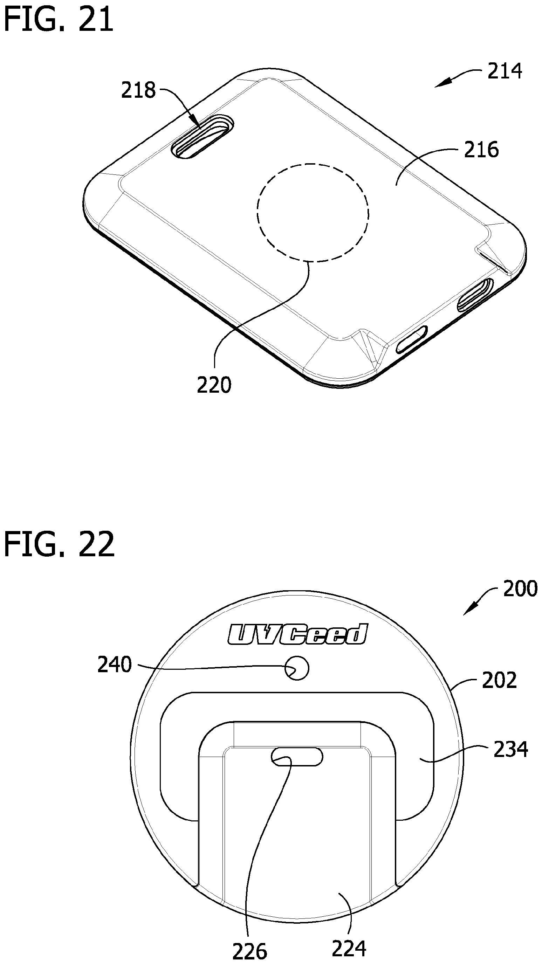

A suitable UV disinfection device for use with the lid adapter is shown in FIG. 21 and indicated at reference numeral 214. This UV disinfection device 214 includes a housing 216 (e.g., generally low-profile housing with a rectilinear footprint), UV source 218 (e.g., UVC source) coupled to the housing (e.g., 275 nm UV light source, such as an LED), a magnet 220 in the housing (not shown), and a microprocessor and memory (not shown) within the housing and wirelessly communicable with an external device (not shown), such as a processor of a mobile device (e.g., mobile phone, hand-held device, etc.) for operating the device. A suitable UV disinfection device is offered for sale by UVCEED. Suitable features, components, devices and/or methods are described above herein, particularly embodiments described in the "UVC Disinfection/Sterilization" disclosure. Additional features which may be incorporated are described in U.S. Serial Nos. 14/609,025, filed January 29, 2025; 16/118,025, filed August 30, 2018; and 17/452,775, filed May 15, 2023. The UV disinfection device may be of other constructions.

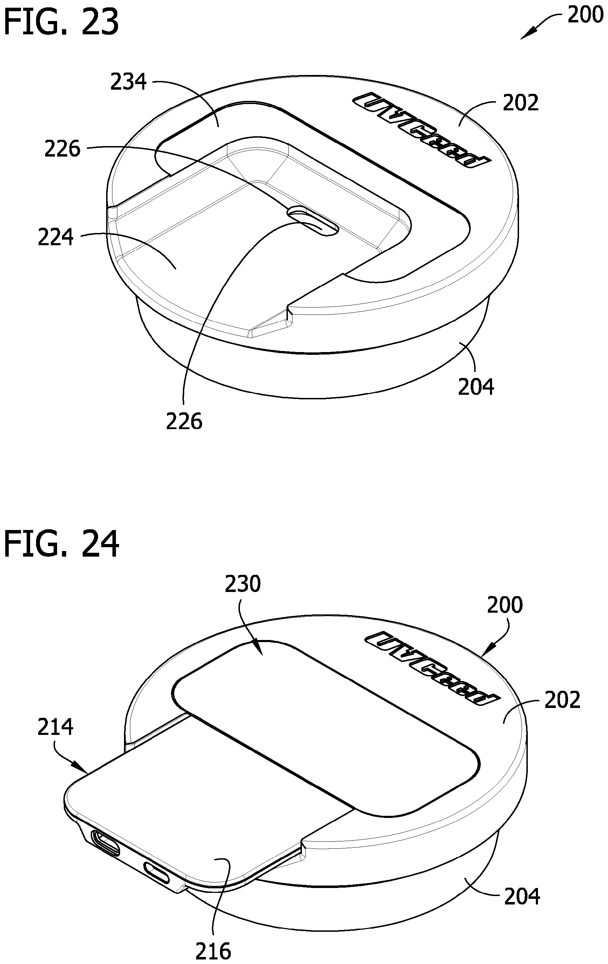

The upper lid body 202 defines a UV device docking area 224 in which the UV disinfection device 214 is receivable. In the illustrated embodiment, the docking area 224 is a recess having size and shape corresponding generally with the size and shape of the UV disinfection device 214 shown in FIGS. 19 and 24. A lower or bottom surface of the docking area 224 (recess) defines a window including an opening 226 extending through the lid body 202. When the UV disinfection device 214 is docked or received in the docking area 224, the UV output (e.g., UV LED) of the device is aligned with the opening 226. In this way, the UV light from the device 214 enters the container 208 through the opening 226 to disinfect the interior of the container.

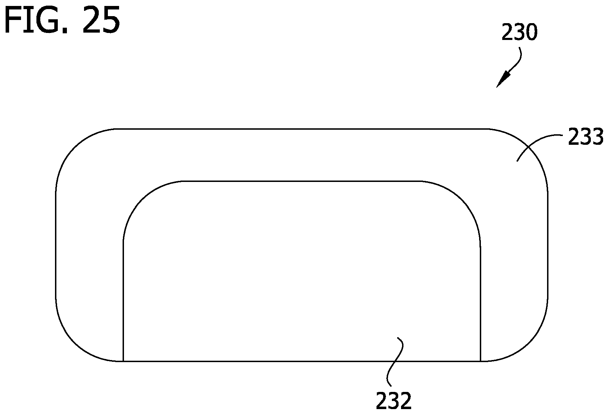

In the example shown in FIGS. 19, 24, and 25, the lid body 202 includes a coupler 230 (e.g., magnetic coupler) configured to magnetically couple the UV disinfection device 214 to the adapter 200. In this example, referring to FIG. 25, the coupler 230 includes a thin strip of magnetic material 232 (e.g., steel) disposed over the docking area 224. An area 234 of the lid body 202 may be slightly recessed to receive an adhesive portion 233 attached to the thin strip of magnetic material 232, so that the magnetic material is attached to the lid body 202 and opposes the lower surface of the docking area 224. Together, the thin magnetic strip 232 and the lower surface of the docking area 224 defines a slot in which the UV disinfecting device 214 is received, as shown in FIGS. 19 and 24. In this example, the UV disinfection device 214 is removably retained in the docking area 224 and slot by the magnetic coupler 230, as can be seen in FIGS. 19 and 24. In another example, the UV disinfection device 214 may be removably coupled to the adapter 200 by snap-fit, friction fit, tongue-and-groove connection, or other ways of mechanically coupling in addition to or as an alternative to the magnetic coupling.

As can be understood from the disclosure, the adapter 200 enables the disinfection device 214 to be removably coupled to the adapter and the container 208 for disinfecting the container. In one or more embodiments, the device 214 may be operated remotely using a smart phone or other hand-held smart device, and the device 214 may be configured to be coupled (e.g., magnetically coupled to the smart phone or other hand-held smart device. As described above, the hand-held smart device may include software for operating the disinfection device 214 when coupled to the adapter 200 for disinfecting the container 208, and the hand-held smart device and the disinfection device may be in remote communication with one another, yet mechanically uncoupled from one another. In one or more embodiments, the disinfection device 214 may include sensors to enable the hand-held device to monitor it for movement when it is coupled to the container. In one or more embodiments, the disinfection device 214 may include sensors to enable the hand-held device to enable the system to calculate the volume of the container using sensors to determine the depth. This data may be used to determine if the appropriate amount of energy is delivered. Different amounts of energy would be required depending if the goal was to disinfect the container, or to treat liquids inside the container to NSF/ANSI 55 standards (Class A and Class B). When using the device 214 to treat liquid or the container, the hand-held smart device may be configured to alert the user via a popup, sound, vibration, or smart watch notification when the treatment level is reached.

The disinfection device 214 may be used to disinfect liquid within the container 208 or an empty container. In one or more embodiments, computer-readable instructions (such as on the hand-held smart device) may perform operations based on the color of the liquid. For example, more opaque liquid may require additional disinfection time and/or dosage. The device may include a camera that capture image data of the liquid and one or more processors determines the color (e.g., opaqueness) of the liquid in the container for determining exposure time and/or dosage.

As shown in FIG. 22, the adapter 200 may include an opening 240 for a straw and/or for drinking the liquid from the container. The opening 240 may include a closure for closing the opening.

Referring to FIG. 23, the window may further include a lens or diffuser (or other optical component, such as such as a collimator, collector, etc.) 244 in registration with the UV light opening 226. Where the optical component 244 is a lens, the lens is configured to widen the angle of the UV light from the device 214, ensuring the entire volume of the water bottle is disinfected using the UV disinfection device. In one or more embodiments, the optical component may be configured to focus the UV light as a convergent beam, divergent beam, collimated beam, or other beam for disinfecting the container and/or liquid inside the container.

In another embodiment, the disinfection device 214 may be removably couplable to another component of a container, other than the lid adapter. The container may have a coupling mechanism that enables the UV from the device to enter the interior of the container. In another embodiment, the container could be for use in a refrigerator such as a drawer or box to hold produce. It could also be a container for personal items to be disinfected. It is also considered that UVC source and supporting electronics could be permanently integrated into the lid.

In one or more embodiments, the device 214 may be configured to run automatically or via a button press.

In another embodiment, a UVC "Pill" could be used to reflect light in a bottle where the inside isn't made of a UVC reflective material, either one or both sides of the pill could be reflective. The "Pill" could be actively controlled with magnets, or could be free flowing in the container.

In one or more aspects, the disclosure is directed to the following numbered paragraphs:

1. An adapter for coupling an ultraviolet (UV) disinfecting device to a container suitable for holding liquid, the adapter comprising:

an adapter body configured to enable the UV disinfecting device to be removably coupled thereto, the adapter body defining a window; and
an adapter coupler connected to the adaptor body, the coupler configured to removably couple with the container to thereby removably couple the adapter to the container,
wherein when the UV disinfecting device is coupled to the adapter body and the adapter coupler is coupled to the container, the window of the adaptor body is alignable with a UV light source of the disinfecting device and configured to enable the UV light source to be in communication with the interior of the container.

2. The adapter set forth in paragraph 1, wherein the adapter body defines a docking area configured to receive the UV disinfecting device to enable coupling of the UV disinfecting device to the adapter body.

3. The adapter set forth in paragraph 2, wherein the window is in registration with the docking area.

4. The adapter set forth in paragraph 2, wherein the docking area defines a recess in the adapter body configured to receive the UV disinfecting device.

5. The adapter set forth in paragraph 4, wherein the adapter body includes a magnetic coupler configured to magnetically couple to the UV disinfecting device when the UV disinfecting device is received in the recess.

6. The adapter set forth in paragraph 5, wherein the magnetic coupler includes a magnetic strip disposed over the recess, wherein the magnetic strip and the recess define a slot sized and shaped to receive the UV disinfecting device.

7. The adapter set forth in paragraph 5, wherein the recess is defined by a surface, wherein the window extends through the surface of the recess.

8. The adapter set forth in paragraph 2, wherein the adapter body includes a magnetic coupler configured to magnetically couple to the UV disinfecting device when the UV disinfecting device is received in the docking area.

9. The adapter set forth in paragraph 1, wherein the adapter coupler is configured to removably couple to an open top of the container.

10. The adapter set forth in paragraph 9, wherein the adapter coupler comprises a resiliently elastic material.

11. The adapter set forth in paragraph 1, in combination with the UV disinfecting device, wherein the UV disinfecting device is removably coupled to the adapter.

12. The adapter set forth in paragraph 11, in further combination with the container, wherein the adapter is removably coupled to the container.

13. The adapter set forth in paragraph 1, wherein the window includes an opening defined by the adapter body, and an optical component in registration with the opening.

14. The adapter set forth in paragraph 13, wherein the optical component comprises a lens.

15. A method of removably coupling a UV disinfection device to a container using the adapter set forth in paragraph 1, the method comprising:

removably coupling the UV disinfection device to the adapter; and
removably coupling the adapter to the container.

16. A lid adapter for coupling an ultraviolet (UV) disinfecting device to a container suitable for holding liquid, the lid adapter comprising:

an adapter body configured to enable the UV disinfecting device to be removably coupled thereto, the adapter body defining a window; and
an adapter coupler connected to the adaptor body, the coupler configured to removably couple to an open top portion of the container to thereby removably couple the adapter to the container,
wherein when the UV disinfecting device is coupled to the adapter body and the adapter coupler is coupled to the container, the window of the adaptor body is alignable with a UV light source of the disinfecting device and configured to enable the UV light source to be in communication with the interior of the container.

17. The lid adapter set forth in paragraph 16, wherein the adapter body defines a docking area configured to receive the UV disinfecting device to enable coupling of the UV disinfecting device to the adapter body.

18. The lid adapter set forth in paragraph 17, wherein the docking area defines a recess in the adapter body configured to receive the UV disinfecting device.

19. The lid adapter set forth in paragraph 18, wherein the adapter body includes a magnetic coupler configured to magnetically couple to the UV disinfecting device when the UV disinfecting device is received in the recess.

20. The lid adapter set forth in paragraph 17, wherein the adapter body includes a magnetic coupler configured to magnetically couple to the UV disinfecting device when the UV disinfecting device is received in the docking area.

Modifications and variations of the disclosed embodiments are possible without departing from the scope of the invention defined in the appended claims.

When introducing elements of the present invention or the embodiment(s) thereof, the articles "a", "an", "the" and "said" are intended to mean that there are one or more of the elements. The terms "comprising", "including" and "having" are intended to be inclusive and mean that there may be additional elements other than the listed elements.

As various changes could be made in the above constructions, products, and methods without departing from the scope of the invention, it is intended that all matter contained in the above description and shown in the accompanying drawings shall be interpreted as illustrative and not in a limiting sense.

## Claims

1. An adapter for coupling an ultraviolet (UV) disinfecting device to a container suitable for holding liquid, the adapter comprising:

an adapter body configured to enable the UV disinfecting device to be removably coupled thereto, the adapter body defining a window; and
an adapter coupler connected to the adaptor body, the coupler configured to removably couple with the container to thereby removably couple the adapter to the container,
wherein when the UV disinfecting device is coupled to the adapter body and the adapter coupler is coupled to the container, the window of the adaptor body is alignable with a UV light source of the disinfecting device and configured to enable the UV light source to be in communication with the interior of the container.

2. The adapter set forth in claim 1, wherein the adapter body defines a docking area configured to receive the UV disinfecting device to enable coupling of the UV disinfecting device to the adapter body.

3. The adapter set forth in claim 2, wherein the window is in registration with the docking area.

4. The adapter set forth in claim 2, wherein the docking area defines a recess in the adapter body configured to receive the UV disinfecting device.

5. The adapter set forth in claim 4, wherein the adapter body includes a magnetic coupler configured to magnetically couple to the UV disinfecting device when the UV disinfecting device is received in the recess.

6. The adapter set forth in claim 5, wherein the magnetic coupler includes a magnetic strip disposed over the recess, wherein the magnetic strip and the recess define a slot sized and shaped to receive the UV disinfecting device.

7. The adapter set forth in claim 5, wherein the recess is defined by a surface, wherein the window extends through the surface of the recess.

8. The adapter set forth in claim 2, wherein the adapter body includes a magnetic coupler configured to magnetically couple to the UV disinfecting device when the UV disinfecting device is received in the docking area.

9. The adapter set forth in claim 1, wherein the adapter coupler is configured to removably couple to an open top of the container.

10. The adapter set forth in claim 9, wherein the adapter coupler comprises a resiliently elastic material.

11. The adapter set forth in claim 1, in combination with the UV disinfecting device, wherein the UV disinfecting device is removably coupled to the adapter.

12. The adapter set forth in claim 11, in further combination with the container, wherein the adapter is removably coupled to the container.

13. The adapter set forth in claim 1, wherein the window includes an opening defined by the adapter body, and an optical component in registration with the opening.

14. The adapter set forth in claim 13, wherein the optical component comprises a lens.

15. A method of removably coupling a UV disinfection device to a container using the adapter set forth in claim 1, the method comprising:

removably coupling the UV disinfection device to the adapter; and
removably coupling the adapter to the container.

FIG. 1

**FIG. 2**

FIG. 3

Lighting Cable Entry / Passthrough

On / Off Switch w/ LED Integration

LED Switching Circuit

Lithium Ion Battery / Approved Charging Circuit

FCC Approved BLE Module w/ GPIO Control

UVC LED(S)

Power Management

# FIG. 4

FIG. 5

FIG. 6

UVA Scanning Figures

UVA Scan

Dim lights and Activate Scan

Activate Scan

UVA Scan

Processing

Recommended Treatment
Areas Found!

Stop

EP 4 520 352 A1

# FIG. 7

FIG. 8

As the device moved
the treatment area was
tracked and new area
starts treatment

**UVC Treatment**

90%    99%    99.9%

Treatment Timer: 00:05

**UVC Treatment**

90%    99%    99.9%

Treatment Timer: 00:15

# FIG. 9

11

body of mask

mask

strap

strap

outer layer

11

# FIG. 10

mask

uvc Device

Outer layer

Inner layer

Air Gap

uvc Device

FIG. 11

mask

13

body of mask

straps

straps

plenum

13

# FIG. 12

FIG. 13

strap

Face Shield

uvc device

Shield

# FIG. 14

Glasses

uvc device

FIG. 15

Ventilator

endotracheal tube

balloon

uvc device

# FIG. 16

uvc Device

uvc Device

uvc Device

uvc Device

# FIG. 17

**FIG. 18**

200

202

234

224

226

204

**FIG. 19**

230

200

214

202

204

## FIG. 20

208

# FIG. 21

# FIG. 22

## FIG. 23

## FIG. 24

**FIG. 25**

**PARTIAL EUROPEAN SEARCH REPORT**

under Rule 62a and/or 63 of the European Patent Convention.
This report shall be considered, for the purposes of
subsequent proceedings, as the European search report

Europäisches Patentamt
European Patent Office
Office européen des brevets

**Application Number**

EP 24 17 2723

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/225514 A1 (MEDKLINN TECH PTE LTD [SG]) 11 November 2021 (2021-11-11) <br> * pg.8, 1.5-7; <br> claim 1; figure 1 * <br> - - - - - | 1,10 | INV. <br> A61L2/10 <br> A61L2/26 |
| X | US 2022/023456 A1 (WOJCZAK SOPHIA [US] ET AL) 27 January 2022 (2022-01-27) <br> * paragraphs [0059], [0064]; claims 12,19; figure 7 * <br> - - - - - | 1,5,6, 13,14 | |
| X | US 8 872 130 B1 (MATTHEWS DAN R [US] ET AL) 28 October 2014 (2014-10-28) <br> * col.11, 1.13-15; <br> claim 1; figure 5 * <br> - - - - - | 1,13,14 | |

**TECHNICAL FIELDS SEARCHED (IPC)**

A61L

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 27 January 2025 | Michel, Marine |

Europäisches
Patentamt
European
Patent Office
Office européen
des brevets

**INCOMPLETE SEARCH
SHEET C**

Application Number

EP 24 17 2723

```
Claim(s) searched incompletely:
      1, 5, 6, 10, 13, 14

Claim(s) not searched:
      2-4, 7-9, 11, 12, 15

Reason for the limitation of the search:

Given the broad scope of the claim 1, the examining division considers
that present claim 1 is not supported by the description because the
generalisation of the following features is unreasonably broad:
The claim unreasonably suggests the use of any type of adapter, which is
not substantiated by the description;
The claim further implies a wide range of possibilities for coupling with
various UV disinfecting devices and containers, which is not adequately
supported in the description.

In view of the arguments regarding the unreasonable breadth of the
claims, especially claim 1, presented above, the combination of the
claimed features appears to be a result of an unreasonably broad
generalisation, so that the present disclosure is insufficient to enable
the skilled person to carry out the claimed invention over its whole
claimed range without carrying out a research program.

The same objections apples for claims 2-15 which are dependent on claim
1-
```

## ANNEX TO THE EUROPEAN SEARCH REPORT
## ON EUROPEAN PATENT APPLICATION NO.

EP 24 17 2723

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2025

| Patent document cited in search report | | Publication date | Patent family member(s) | | Publication date |
|---|---|---|---|---|---|
| WO 2021225514 | A1 | 11-11-2021 | NONE | | |
| US 2022023456 | A1 | 27-01-2022 | US | 2022023456 A1 | 27-01-2022 |
| | | | WO | 2022020121 A1 | 27-01-2022 |
| US 8872130 | B1 | 28-10-2014 | US | 8872130 B1 | 28-10-2014 |
| | | | US | 9346687 B1 | 24-05-2016 |

For more details about this annex : see Official Journal of the European Patent Office, No. 12/82

**REFERENCES CITED IN THE DESCRIPTION**

**Patent documents cited in the description**

- US 02515218
- US 11366618
- US 11802518
- US 94118501
- US 025152
- US 113666
- US 60902525
- US 16118025 B
- US 17452775 B